# EUROPEAN PATENT APPLICATION

(11) **EP 4 650 812 A1**
(43) Date of publication of application: **19.11.2025**
(21) Application number: 25173074.3
(22) Date of filing: 29.04.2025
(51) Int. Cl.: G01R 33/565, A61B 5/11, G01R 33/567

(54) **MAGNETIC RESONANCE IMAGING APPARATUS WITH MEANS FOR ADAPTIVE CORRECTION OF BODY MOVEMENTS**

(30) Priority: 15.05.2024 JP 2024079687
(71) Applicant: FUJIFILM Corporation, Tokyo 106-8620 (JP)
(72) Inventor: HAMADA, Kota, Tokyo, 106-8620 (JP); IKEGAWA, Ayaka, Tokyo, 106-8620 (JP)
(74) Representative: Dehns Germany Partnerschaft mbB

(57) **Abstract**

Provided are a unit capable of more detailed analysis of a body movement occurring during an MRI examination and an MR image in which body movement correction with high accuracy is performed by the unit.

An MRI apparatus includes a processor configured to perform body movement processing. The processor is configured to set, in a video of an imaging device that monitors a body movement, a region (target region) for monitoring a body movement with respect to an examination target, monitor a movement of a subject between the target region and a region outside the target region along with a retention time in each region, and accurately specify a part of measurement data to be a target of body movement correction.

## Description

### CROSS-REFERENCE TO RELATED APPLICATIONS

The present application claims priority to Japanese Patent Application No. 2024-079687, filed May 15, 2024. Each of the above application(s) is hereby expressly incorporated by reference, in its entirety, into the present application.

### BACKGROUND OF THE INVENTION

### 1. Field of the Invention

The present invention relates to a magnetic resonance imaging apparatus (hereinafter, referred to as an MRI apparatus), and particularly to a processing technology for body movement of a subject during an examination using the MRI apparatus.

### 2. Description of the Related Art

In an MRI apparatus, a subject is placed in an examination space where a static magnetic field is generated, a gradient is applied to the static magnetic field, position information is added to a nuclear magnetic resonance signal (hereinafter, referred to as an NMR signal or an echo signal), and k-space data required for image reconstruction is collected.

In a case where the subject has moved during the examination, there is a problem in that the position information is not appropriately added by the gradient magnetic field, and a positional deviation or an artifact occurs in the reconstructed image. Body movements include periodic body movements with relatively small displacements, such as respiratory movements and pulsation, and irregular body movements, such as sudden movements of the subject, and particularly, the latter, the irregular body movements are problematic. As processing on an apparatus side with regard to the body movement of the subject during the examination, correction is performed using a method of removing a part of the k-space data collected when a body movement occur and zero-filling or estimating the missing data (refer to, for example, JP1997-028689A (JP-H09-028689A)). The correction corresponding to the body movement is referred to as body movement correction. In addition, JP2018-050668A describes that in an X-ray CT apparatus, an imaging range is set based on body movement data of a subject, and a scan is stopped in a case where a body movement range exceeds the imaging range during the scan.

On the other hand, as a method of detecting a body movement during an examination, various methods are known, such as a method using a video from a camera (surveillance camera) for monitoring a subject installed in or near an examination space, and a method of collecting an NMR signal for body movement detection (called a navigator echo) in addition to an NMR signal for image generation collected from the subject, and detecting a body movement from the navigator echo. In the case of the body movement correction, the information from the body movement detection units is processed in the apparatus to determine whether or not the body movement that affects the imaging has occurred, and the body movement correction as described above is performed.

### SUMMARY OF THE INVENTION

The technology described in JP1997-028689A determines whether or not a magnitude of the body movement is within an allowable range, whether or not body movement correction is necessary, and the like. However, details of the magnitude, direction, and duration of the body movement cannot be analyzed. This can lead to problems such as excessive body movement correction (overcorrection) resulting in image blurring, or conversely, insufficient body movement correction, resulting in inability to completely eliminate artifacts caused by a body movement. JP2018-050668A is a technology that focuses on the X-ray CT apparatus, where processing performed in a case where the body movement range exceeds an allowable range is issuing a warning or stopping the scan, and a relationship between the body movement and the body movement correction is not considered in the first place.

An object of the present invention is to provide a unit capable of analyzing a body movement occurring during an examination in more detail and to provide an image subjected to body movement correction with high accuracy.

In order to solve the above-described problem, an MRI apparatus according to an aspect of the present invention comprises a processor configured to perform body movement processing. The processor is configured to set, in a video of an imaging device that monitors a body movement, a region (target region) for monitoring a body movement with respect to an examination target, monitor a movement of a subject between the target region and a region outside the target region along with a retention time in each region, and accurately specify a part of measurement data to be a target of body movement correction.

That is, the MRI apparatus according to the aspect of the present invention comprises: an imaging unit that collects a nuclear magnetic resonance signal generated from a subject; an image generation unit that generates an image using the nuclear magnetic resonance signal; and a processor that is configured to acquire a video from an imaging device which detects a body movement of the subject and process body movement information of the subject. The processor is configured to set a first imaging region for monitoring an examination area of the subject in the video, determine at least one of presence or absence of the body movement, duration of the body movement, a direction of the body movement, or a magnitude of the body movement based on whether or not the examination area is shifted from the first imaging region to an outside of the first imaging region, and control body movement correction by the image generation unit.

Here, the "duration of the body movement" includes not only a time during which the movement of the examination area is continued but also a time during which the examination area remains at a position different from an initial position even though the movement is stopped.

According to the aspect of the present invention, by monitoring the movement and a retention time of the subject outside the target region, detailed information such as the magnitude, the direction, and the time of the body movement can be acquired, and appropriate body movement correction can be performed based on the information. As a result, it is possible to prevent blurring of the image due to overcorrection, artifacts due to improper correction, and the like.

### BRIEF DESCRIPTION OF THE DRAWINGS

Fig. 1 is a diagram showing an overall overview of an MRI apparatus.
Fig. 2 is a diagram showing an example of disposition of a body movement detection unit in the MRI apparatus.
Fig. 3 is a diagram showing a configuration of a processor of the MRI apparatus.
Fig. 4 is a diagram showing a flow of body movement processing by the processor.
Fig. 5 is a diagram showing a flow of the body movement processing of Embodiment 1.
Figs. 6A to 6D are diagrams for describing target region setting in Embodiment 1, in which Figs. 6A to 6D are diagrams, each showing a specific example of a target region.
Figs. 7A and 7B are diagrams illustrating body movement processing of Embodiment 1, in which Fig. 7A shows a case where a signal acquisition order is a sequential order and Fig. 7B shows a case where the signal acquisition order is a centric order.
Fig. 8 is a diagram showing a flow of body movement processing of Embodiment 2.
Figs. 9A to 9C are diagrams for describing target region setting in Embodiment 2, in which Figs. 9A to 9C are diagrams, each showing a specific example of a target region.
Figs. 10A and 10B are diagrams illustrating the body movement processing of Embodiment 2.

### DESCRIPTION OF THE PREFERRED EMBODIMENTS

Hereinafter, embodiments of the present invention will be described with reference to the accompanying drawings.

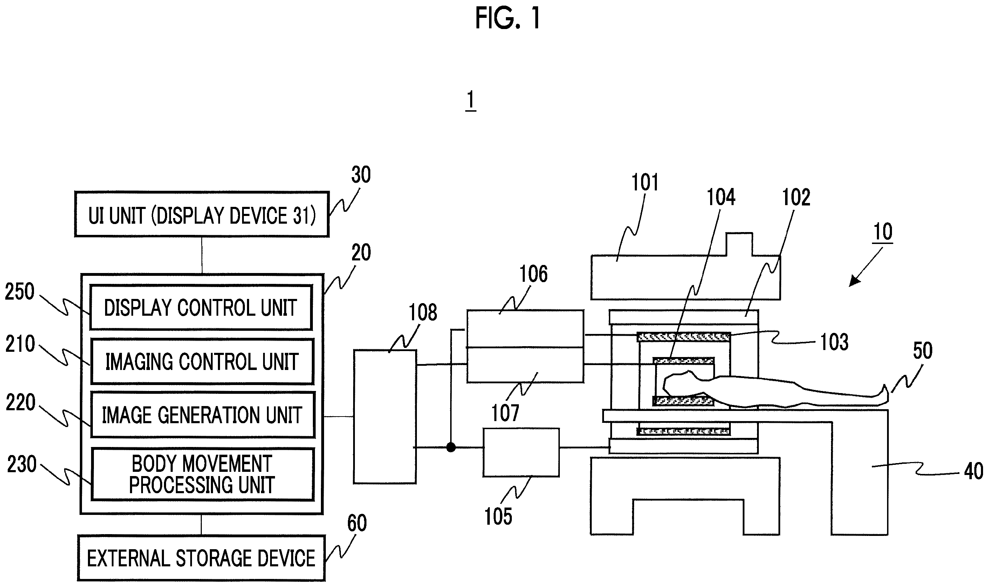

Fig. 1 is a diagram showing an overall overview of an MRI apparatus to which the present invention is applied. The MRI apparatus mainly includes an imaging unit 10 that provides an examination space, induces NMR in an examination area of a subject 50 placed in the examination space, and collects an NMR signal generated from the examination area, and a processor 20 that has functions of a calculation unit that reconstructs an image using k-space data consisting of the NMR signal collected by the imaging unit 10 and a control unit that controls the entire apparatus including the imaging unit 10.

The functions of the imaging unit 10 are similar to those of a known MRI apparatus, and detailed description thereof will be omitted in this specification. However, as shown in the figure, the imaging unit 10 comprises a static magnetic field magnet 101 that generates a uniform magnetic field (static magnetic field) in the examination space in which the subject 50 is placed, a gradient magnetic field coil 102 that applies a gradient magnetic field to the static magnetic field, an RF transmission coil 103 that applies a high-frequency magnetic field to excite nuclei of atoms constituting a tissue of the subject, and an RF reception coil 104 that receives an NMR signal generated by the subject, in which the gradient magnetic field coil 102, the RF transmission coil 103, and the RF reception coil 104 are connected to a gradient magnetic field power supply 105, a transmitter 106, and a receiver 107, respectively. Operations of the gradient magnetic field power supply 105, the transmitter 106, and the receiver 107 are controlled by a sequencer 108. The sequencer 108 determines a pulse sequence for each scan using a set pulse sequence type and imaging conditions (imaging parameters) such as imaging parameters, and controls each component of the imaging unit 10 to operate in accordance with the determined pulse sequence and collect an echo signal (k-space data) necessary for image reconstruction. Since functions and operations of each component in a case where the imaging unit 10 collects the k-space data are similar to those of a general MRI apparatus, detailed description thereof will be omitted here.

The static magnetic field magnet 101, the gradient magnetic field coil 102, and the RF transmission coil 103 are housed in a gantry, and the subject 50 is positioned in the examination space in the gantry in a state of being laid on a bed device 40 with the RF reception coil 104 attached to the examination area.

The processor 20 functions as the control unit that controls imaging via the sequencer 108 and that controls operations of the entire apparatus, and functions as the calculation unit that reconstructs an image of the subject using the echo signal collected by the imaging unit 10 or performs a calculation such as correction on the k-space data before reconstruction or an image after reconstruction. The processor 20 of the present embodiment has a function of collecting and processing body movement information generated in the subject 50 during an examination, for example, a magnitude and duration of a body movement, and associating the body movement information with a scan in progress (imaging) to be presented to a user, in addition to the functions of the control unit and the calculation unit described above.

The processor 20 can be configured with one or a plurality of computers 200 comprising a CPU, a GPU, or both thereof and a memory, and performs various calculations using the NMR signal and controls of the entire apparatus as described above. However, some of the functions of the processor 20 may be implemented by a programmable IC such as an ASIC or a PGFA, and the processor 20 and the programmable IC are collectively referred to as the processor 20.

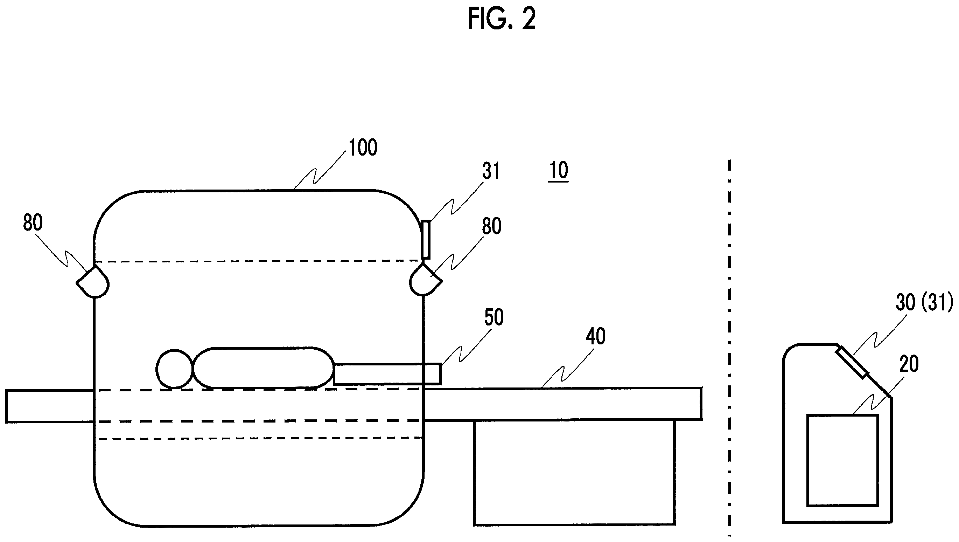

A UI unit 30 comprising a display device 31 or an output device (not shown) for performing a communication with the user, an external storage device 60, and the like are connected to the processor 20. As shown in Fig. 2, the display device 31 may be provided in a console operated by the user, or in a plurality of locations such as near or on a surface of a gantry 100 that provides the examination space. In addition, the processor 20 may be connected to an external database such as PACS or another image processing device via a known network connection.

In addition, in the MRI apparatus of the present embodiment, a body movement detection unit such as a surveillance camera is installed inside or in the vicinity of the examination space to allow the processor 20 to perform detailed analysis on the body movement of the subject occurring during the examination, and enable appropriate body movement correction. For example, as shown in Fig. 2, a plurality of surveillance cameras 80 may be installed at two or more locations, such as at two openings of the gantry 100, on an entrance side and an opposite side thereof. In this case, the processor 20 processes videos from the plurality of surveillance cameras. In addition to the surveillance camera 80, information after using the NMR signal (navigator echo) as the body movement detection unit may be used.

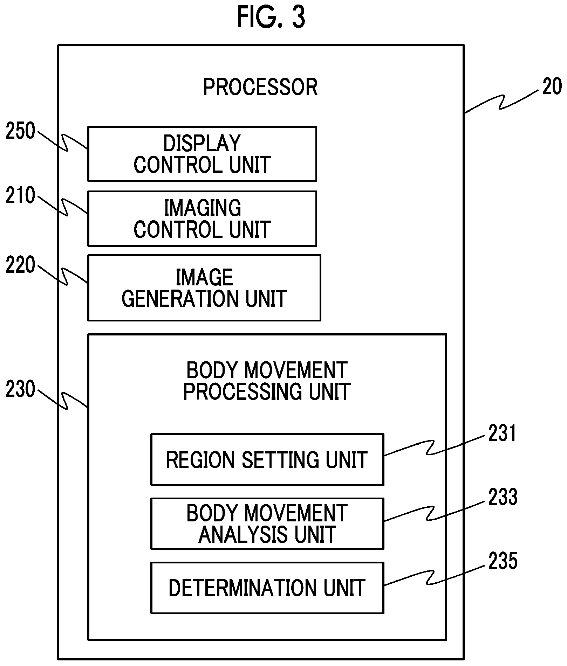

Fig. 3 shows a configuration example of the processor 20 of the present embodiment. As shown in the figure, the processor 20 comprises an imaging control unit 210 that controls the imaging unit 10 via the sequencer 108, a display control unit 250 that controls a display of an image and a GUI on the display device of the UI unit 30, an image generation unit 220 that generates an image of the subject using the k-space data consisting of the NMR signal, and a body movement processing unit 230. In addition, although not shown in the figure, a functional unit included in a known MRI apparatus, such as a functional unit that performs calculations using reconstructed images, image correction, and the like may be provided.

The body movement processing unit 230 comprises a region setting unit 231 that sets a region (target region) for monitoring the body movement of the subject, a body movement analysis unit 233 that analyzes a movement (magnitude, direction, retention time, and the like) of the subject entering and leaving the target region using information (referred to as body movement information) from the body movement detection unit, and a determination unit 235 that determines subsequent processing by the calculation unit or the control unit based on a result of the analysis by the body movement analysis unit 233.

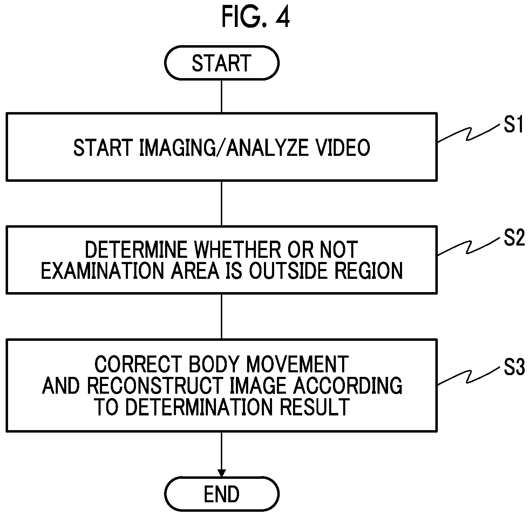

Fig. 4 shows an outline of processing of the MRI apparatus of the present embodiment with the above configuration. As shown in the figure, in a case where imaging is started, the body movement analysis unit 233 analyzes the video from the surveillance camera 80 (S1), the determination unit 235 determines whether or not the examination area of the subject is within a region set in advance based on an initial position of the examination area and whether or not the time that the subject is within the region or outside the region is within a predetermined time (S2), and a content of body movement correction is determined according to a determination result (S3).

Prior to the analysis by the body movement analysis unit 233, the MRI apparatus of the present embodiment sets a region (referred to as a target region) for monitoring the body movement from the initial position of the examination area of the subject in the camera video in a case of starting the imaging, monitors a retention time of the examination area inside and outside the target region, and determines the content of the body movement correction accordingly. A method of setting the region and the content of the body movement correction according to the determination result will be described in detail in the following embodiment.

According to the present embodiment, instead of estimating the presence or absence and the magnitude of a body movement from movements of feature points of interest as in the related art, a target region (a two-dimensional extent) is set to include the examination area, and a body movement is determined based on whether the examination area remains in the target region, so that the body movement can be accurately determined even for movements in various directions. As a result, it is possible to reduce overcorrection or undercorrection of the body movement correction.

Hereinafter, a specific embodiment of the processing by the processor of the present embodiment will be described.

### Embodiment 1

In the present embodiment, the processor sets a single region including the examination area at the start of the examination in the camera image as a target region to be monitored, monitors movement of the examination area from the target region to an outside of the target region and a time (retention time) in a case where a part of the examination area is outside the target region, and controls the content of subsequent body movement correction according to a monitoring result.

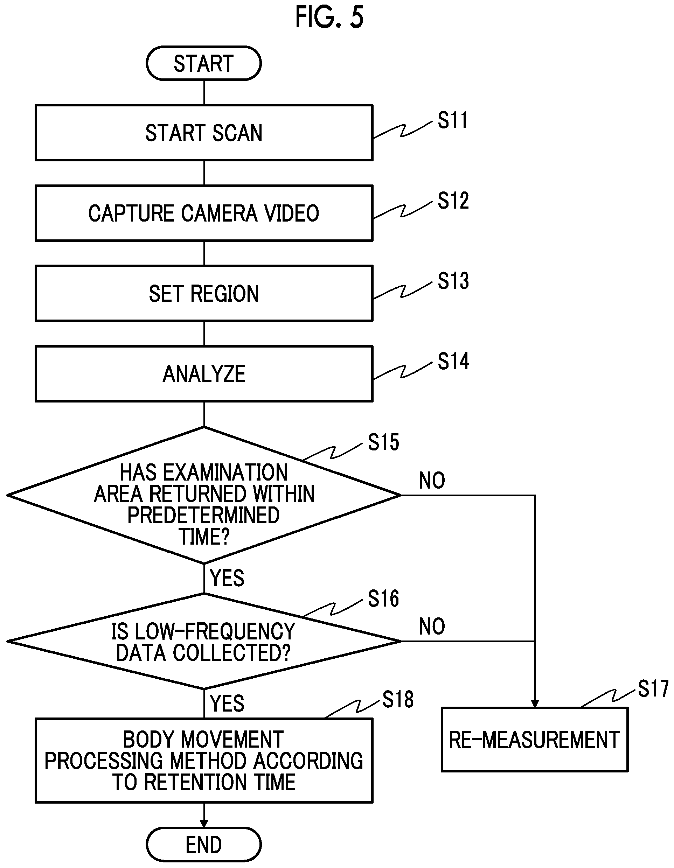

A flow of the processing of the present embodiment will be described with reference to a flowchart of Fig. 5.

### S11

First, in a case where a scan task (pulse sequence) to be performed in one imaging or a scan parameter is determined according to a preset examination protocol or the like, the imaging control unit 221 controls the imaging unit 10 to start imaging (S11). There are one or a plurality of scans included in one imaging, and a type of imaging (for example, T1-weighted imaging, T2*-weighted imaging, diffusion-weighted imaging) and the imaging method (sampling method) performed in each scan are optional and not particularly limited. Here, as an example, a flow of performing one scan for collecting k-space data necessary for reconstructing one image or one set of images is shown.

In a case where navigator data is used in combination as the body movement detection unit, the imaging unit 10 performs imaging by adding a sequence for collecting navigator echoes to a pulse sequence for acquiring an image of the subject. As the sequence for collecting the navigator echoes, various methods are known, such as a method of adding a step of generating an RF pulse to collect navigator echoes in a pulse sequence for main imaging, and a method of performing a navigator sequence separately from a pulse sequence for main imaging, and any of these methods can be adopted.

### S12 and S13

The body movement processing unit 230 captures in the video from the surveillance camera 80 before and after the start of the imaging (S12). Before the scan (pulse sequence) is started, first, the region setting unit 231 sets a region of the examination area at the initial position (hereinafter, referred to as a target region) using the video of the subject (S13). The target region is a region set to monitor the body movement of the examination area, and a size and shape thereof are not particularly limited and can be changed depending on the examination area. That is, the target region can have any shape such as a circular shape, an elliptical shape, a rectangular shape, and a trapezoidal shape according to the shape of the examination area, and the size of the region is set, for example, to cover the examination area according to the size of the examination area. For example, the setting may be performed by displaying an image (video) in which the examination area is shown on the display device 31 of the UI unit 30, determining a target region having a predetermined size and shape by the user through an operation using a cursor or the like on a screen of the display device 31, and setting the target region as the target region (a fixed region in the video of the position shown by the surveillance camera) in the video by the region setting unit 231, or the region setting unit 231 may specify a region of the head or the examination area on which the reception coil is mounted in the camera image using an image recognition or feature amount extraction algorithm based on the information of the examination area included in the examination protocol, and set the target region to include the region of the examination area.

As described above, the size and shape of the target region may be set by the user or may be automatically set by the apparatus based on the size, direction, and the like in which the body movement is allowed, according to an imaging area or a type of imaging, as will be described later.

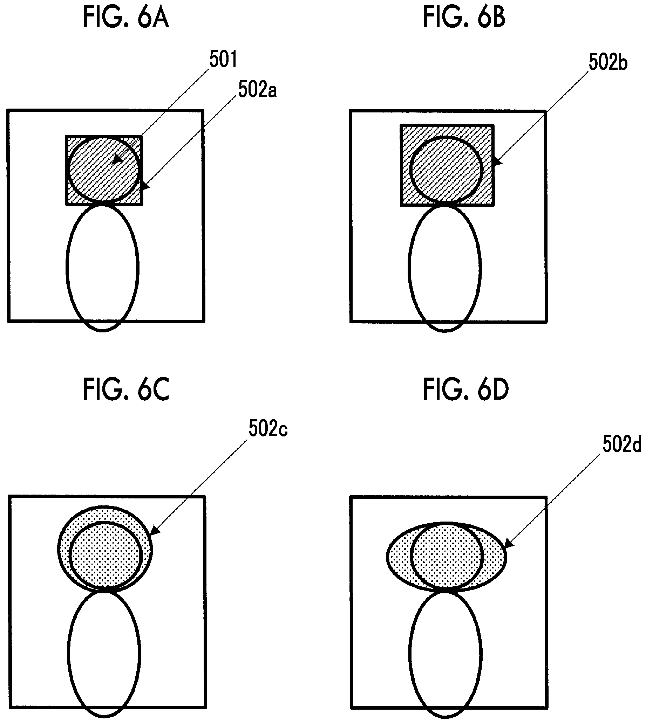

Figs. 6A to 6D show examples in a case where the examination area is the head.

Fig. 6A is an example in which, for a region (examination area region) 501 recognized as the head on the camera image, a rectangular region of which vertical and horizontal lengths are set to a maximum width in a vertical direction and a maximum width in a horizontal direction of the examination area region and that is a region inscribing the region 501 is set as a target region 502a.

Fig. 6B is an example in which a region wider than the region 502a is set as a target region 502b, Fig. 6C is an example in which a circular target region 502c is set, and Fig. 6D is an example in which an elliptical target region 502d is created by widening a width of the circular region 502c shown in Fig. 6C in the horizontal direction. These are merely examples, and a shape of the target region, a clearance in each direction with respect to the examination area, and the like can be variously changed.

For example, regarding a clearance of the target region 502 (502a to 502d are collectively referred to as 502) for the region 501, the presence or absence and the size of the clearance may be changed according to a tolerance for the body movement. In a case where the tolerance for the body movement is small, that is, in a case of imaging in which even a slight body movement cannot be allowed, the clearance is set to be small, and the movement of the examination area that protrudes from the target region 502 can be accurately detected. In a case of imaging in which the tolerance for the body movement is relatively large, the target region 502 is set by providing a clearance (about several mm) between the target region 502 and the region 501 as in the example shown in Fig. 6B or Fig. 6D.

The tolerance for the body movement may vary depending on the imaging area and the type of imaging. For example, it is considered that the head, where fine structures are an issue, has a lower tolerance for body movement than the trunk. In addition, with regard to the head, movement in a left-right direction may be allowed to some extent, but movement in an up-down direction is considered to be accompanied by a large body movement and may be set to have a low tolerance. In that case, as in the example shown in Fig. 6D, the size or presence or absence of the clearance may be different between the left-right direction and the up-down direction.

Examples of the types of imaging that may have different tolerances for body movement include a sampling method in a case of collecting data on the k-space and types of scans. For example, sampling methods such as radial sampling or PROPELLER that samples k-space radially from the center, and spiral scans that sample k-space in a spiral pattern, are sampling methods robust to body movement and thus have a high tolerance for body movement. In addition, scans using scout imaging for positioning the subject in the examination space have a relatively high tolerance for body movement. Alternatively, a GUI for allowing the user to set a desired tolerance for body movement in advance may be displayed on the UI unit 30, and the user may set information regarding the tolerance for body movement in advance in consideration of the type of imaging and the like.

In a case where the region setting unit 231 sets the region, for example, a rectangle or a circle is prepared as a basic shape, and the region setting unit 231 can automatically set the region or receive a user selection in accordance with a feature of the shape of the examination area. In addition, even in a case where the clearance is set based on the tolerance for body movement, as in the selection of the shape, in a case where the information regarding the tolerance for body movement is obtained in advance, the region setting unit 231 may automatically set the clearance based on the information or receive a user designation to set the clearance through the UI unit 30.

### S14

In a case where the target region is set, the body movement analysis unit 233 analyzes the video sent from the surveillance camera 80 for each frame, and determines whether the examination area remains in the target region 502 or is outside of the target region (that is, whether a part of the examination area is shifted to the outside of the target region) by using a known image recognition technology, or a technology such as optical flow or non-rigid transformation (S14). In a case where it is determined that the examination area protrudes from the target region, the body movement analysis unit 233 records a time of a frame of the video at which the protrusion occurs. The body movement analysis unit 233 continuously determines whether the examination area is within the target region 502 or protrudes from the target region 502, records the time of the frame at which the examination area returns to the target region 502 after the examination area protrudes from the target region 502, and sets the time during which a part of the examination area is outside of the target region as a retention time.

The body movement analysis unit 233 continues to capture (S12) and analyze (S13) the camera video until one scan is ended, that is, until all the planned k-space data is collected, unless a situation arises in which the scan has to be stopped.

### S15 to S18

The determination unit 235 determines subsequent processing in consideration of the retention time and the arrangement of the k-space data collected during the retention time on the k-space. Regarding the body movement of the examination area, there are cases where the examination area returns to the target region during the scan after the examination area moves out of the target region, and cases where the examination area does not return to the target region. First, in a case where the examination area returns to the target region, it is determined whether or not the examination area returns to the target region within a predetermined time (S15).

The predetermined time is determined by whether or not the number of pieces of data that can be corrected is collected, and varies depending on a repetition time (TR) of an imaging mode and a method of the body movement correction that can be adopted in subsequent image processing. The predetermined time can be set in advance accordingly. By appropriately setting the predetermined time according to the type of scan, the method of the body movement correction, and the like, it is possible to avoid overcorrection or undercorrection.

For example, in a case where the TR is long, an interval between the data collection of one line of the k-space and the data collection of the next line becomes long. Therefore, even in a case where the retention time is set to be relatively long, the number of lines collected during the retention time is smaller than that in the scan with a short TR. Therefore, the predetermined time may be set to be long.

In addition, for example, in a case where zero-filling is adopted as the body movement correction or in a case of iterative reconstruction in which data estimation is performed by repeated calculations, as long as at least about 80% of the number of phase encodes of the k-space data can be collected, that is, as long as the retention time is equal to or shorter than a time needed for collecting data of 20% of the number of phase encodes, image reconstruction by zero-filling is possible, and thus the time is set as a predetermined time. In addition, as the method of the body movement correction, in a case of a half scan, image reconstruction can be performed in a case where one of two pieces of high-frequency data sandwiching a low-frequency region of the k-space can be collected, it is also possible to set a time needed for collecting 60% to 70% of data as the predetermined time and to set a shorter time (50% or more) as the predetermined time on a condition that one of the pieces of high-frequency data is collected. However, in the image reconstruction using a small number of pieces of data, an SN ratio deteriorates. Therefore, in the imaging aiming at a high SN ratio, it is preferable to set the predetermined time to be short, and in this case, a target SN may be considered in the setting of the predetermined time.

In a case where the examination area does not return to the target region within the predetermined time (S15), the determination unit 235 determines that it is difficult to perform significant image reconstruction even in a case where the body movement correction is performed and it is necessary to perform re-measurement (S17), and passes the result to the imaging control unit 210 and/or the display control unit 250. The imaging control unit 210 may automatically perform the re-measurement based on the determination result of the determination unit 235 that the re-measurement is necessary, or the display control unit 250 may notify the user by displaying a message such as "The subject moved, re-measurement is necessary" or a mark indicating that there is a body movement, on the display device 31. In response to this notification, the user may send a command to perform the re-measurement or a command for a re-measurement range to the imaging control unit 210 via the UI unit 30.

On the other hand, in a case where the retention time is within the predetermined time (S15), the determination unit 235 further determines whether or not the k-space data collected during the retention time is in the low-frequency region of the k-space (S16). For example, even in a case where the retention time is a time needed for collecting 20% of the data, in a case where the k-space data collected during the retention time is the low-frequency data, reconstruction of a diagnostically valuable image cannot be performed, and re-measurement of the low-frequency data is necessary. In this case, the determination result indicating the need for re-measurement is sent to the imaging control unit 210 or the display control unit 250 as in the above-described case.

As a result of the determination (S15, S16), in a case where the retention time is within the predetermined time and the required low-frequency data is collected, the processor 20 (determination unit 235) controls an image reconstruction unit to perform image reconstruction by adopting a body movement correction method corresponding to the set predetermined time, for example, zero filling.

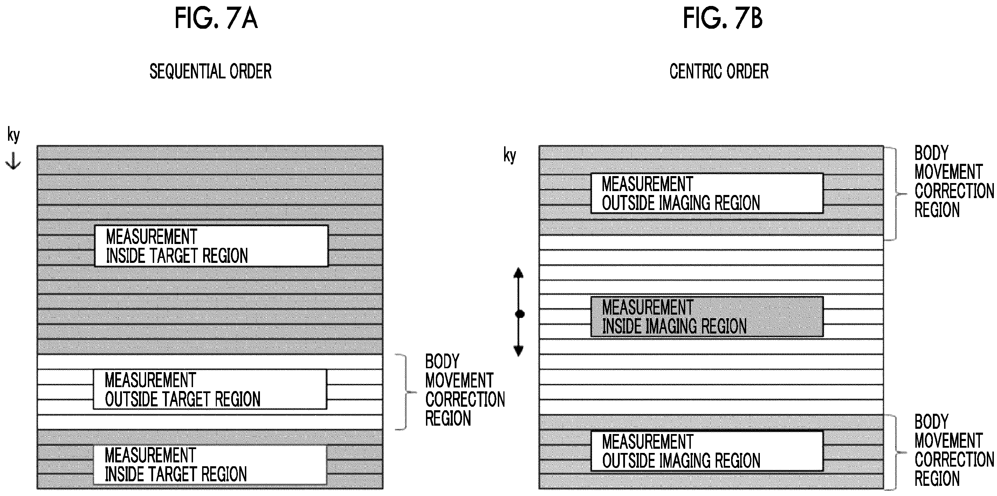

The body movement correction method may be selected in consideration of a k-space data collection order (ordering) in addition to the set predetermined time and the ratio of the data collected during the predetermined time to the k-space data. Although the data collection order that can be adopted in the present embodiment is not particularly limited, two representative examples of the data collection order are shown in Figs. 7A and 7B. Fig. 7A is an example of a sequential order in which high-frequency data is collected from one side of k-space to the other side via zero-encoding, and Fig. 7B is an example of a centric order in which data is alternately collected from the center of the k-space toward both sides in order.

In Fig. 7A, for example, in a case where data collection is performed outside the target region while collecting data from the low-frequency data to the high-frequency data on the other side, in a case where the data collection outside the target region is within a predetermined time and the data collected during the time period is not the low-frequency data including the center of the k-space and is within 20% of the k-space, the data collected outside the target region can be set as a target for the body movement correction, and the image reconstruction using zero-filling or half scan can be performed. In this ordering, the high-frequency data on one side is collected, so that half scan reconstruction can be performed.

In a case of the centric order in which data is collected from the center to the high-frequency region of the k space shown in Fig. 7B, the low-frequency data is first collected. Therefore, a body movement occurs during the collection of the high-frequency data, and there is a high probability that the high-frequency data is a target for body movement correction. In such a case, the image reconstruction using the half scan cannot be performed, so that body movement correction of performing image reconstruction using the zero-filling of the high-frequency data on both sides is adopted. In addition, although not shown, depending on the ordering, lines (for example, an n-th line, an (n + 3)-th line, an (n + 6)-th line, and the like) that are discontinuous in a phase-encoding direction of the k-space data may be a target for body movement correction. In such a case, it is also possible to perform iterative reconstruction in which data estimation is performed by repeated calculations.

As described above, according to the present embodiment, the target region in which the body movement is allowed for the examination area is set for the video from the surveillance camera, the protrusion of the examination area from the target region to the outside and the retention time outside the target region are analyzed, and the processing such as whether or not the body movement correction is necessary, the selection of the body movement correction method, and whether or not the re-measurement is necessary is determined based on the analysis result. Therefore, compared to the body movement correction performed simply based on the magnitude of the body movement, it is possible to grasp two-dimensional body movements, to improve the accuracy of the body movement correction, and to reduce the probability of overcorrection or undercorrection.

In particular, the above-described effect can be enhanced by appropriately setting the clearance between the examination area and the target region according to the examination area or the type of imaging.

### Embodiment 2

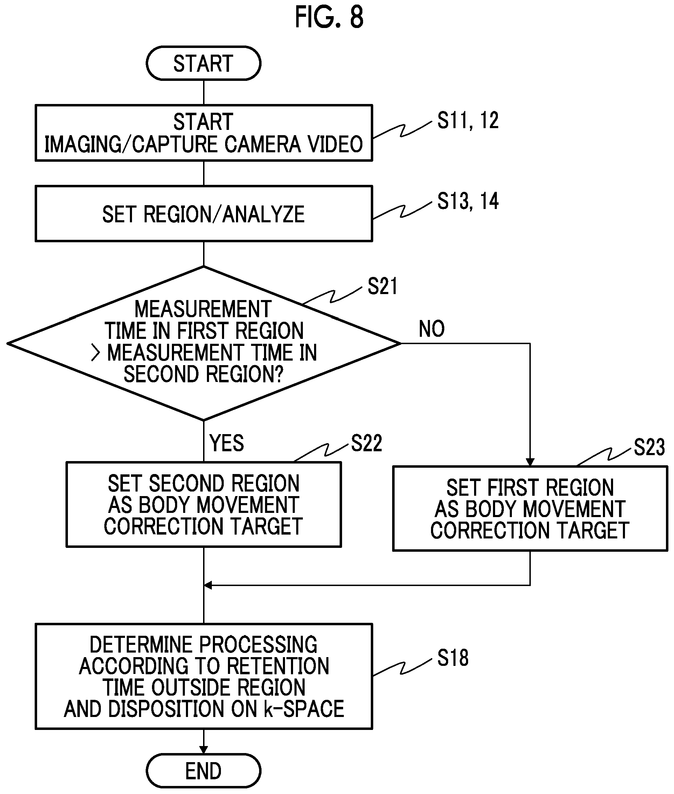

The present embodiment is characterized in that a second region for monitoring the body movement is set outside the target region. Hereinafter, processing of the present embodiment will be described with reference to a flowchart of Fig. 8. In Fig. 8, the same processes as those in Fig. 5 are denoted by the same reference numerals, and the overlapping description will be omitted.

In the present embodiment, in a case where the imaging is started (S11), the video from the surveillance camera 80 is captured prior to scanning (S12), and a region including the examination area is set with a position of the examination area at that time as an initial position (S13). In Embodiment 1, a single region having a predetermined shape to surround the region 501 of the examination area is set as the target region 502. However, in the present embodiment, the region setting unit 231 sets a second region outside a first region (for example, the region 502 shown in Figs. 6A to 6D). The first region is a region of any shape including the examination area of the subject, and can be set in the same manner as the region 502 of Embodiment 1. The second region is a region located outside the first region, and may be a region surrounding an entire outer periphery of the first region, or may be provided close to a part of the outer periphery, for example, both sides or one side in the vertical direction, or both sides or one side in the horizontal direction. In addition, a third region may be further provided outside the second region.

Furthermore, it is also possible to provide a gap between the first region and the second region. A width of the gap can be adjusted according to the tolerance for the body movement, similarly to the clearance between the region 501 of the examination area and the target region 502 in Embodiment 1. That is, the presence or absence of the gap and the width of the gap can be adjusted depending on whether the sampling method (radial sampling or the like) having a relatively large tolerance for body movement is used or the type of scan is used.

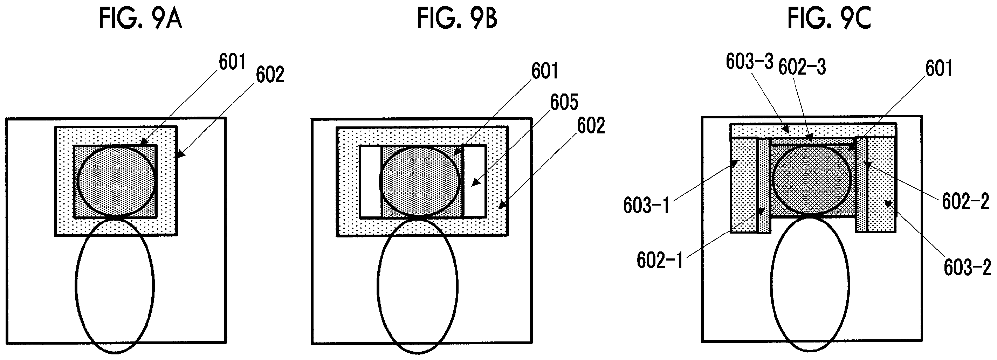

Figs. 9A to 9C show setting examples of the second region. Fig. 9A is an example in which a second region 602 is set to be in contact with a rectangular first region 601, and Fig. 9B is an example in which a gap 605 is set between the first region 601 and the second region 602 of Fig. 9A. Here, the gap 605 is provided only on both sides of the first region 601 in the horizontal direction, and the second region 602 is in contact with the first region 601 in the vertical direction. This applies in a case where the tolerance for body movement in the horizontal direction is relatively large.

Fig. 9C is an example in which a plurality of second regions 602 are set outside the first region 601, and a third region 603 is further set outside the second regions 602. In the shown example, second regions 602-1, 602-2, and 602-3 having relatively narrow widths are set on both left and right sides and an upper side of the first region 601, and third regions 603-1, 603-2, and 603-2 are set outside the second regions. Widths of the second region and the third region may be adjusted according to the tolerance for body movement, similarly to the gap between the regions. In this way, by setting the second regions 602-1, 602-2, and 602-3 in a plurality of directions, information on the direction of the body movement can be obtained, and by setting a plurality of regions (the second regions and the third regions) toward the outside, information on the magnitude of the body movement can be obtained.

For example, it is possible to obtain information on whether the examination area has moved to the right or to the left, whether there was a large body movement exceeding the width of the second region, or whether there is a body movement small enough to remain in the second region. Furthermore, by using the information on the width and the retention time of the region, more detailed information on the body movement, for example, whether the body movement is a relatively gentle movement or an instantaneous movement, or whether the body movement is a movement that immediately returns or a movement that does not return can be obtained. Based on such information, the determination unit 235 can perform appropriate determinations for subsequent processing. In addition, it is also possible to present the information regarding the magnitude and the direction of the body movement obtained by the body movement processing unit 230 to the user or to provide feedback to the subject during the examination, thereby enabling the subject to move to return to the posture or to prompted to return to the original posture on his/her own.

A method of presenting information is not limited, and various methods can be adopted, for example, superimposing an image mark of a reference position imitating the subject and an image mark in a state in which a position deviation occurs in an identifiable manner to be displayed on a screen, and issuing an audio warning such as "You have moved to the right, please return to position".

As described above, after the region setting unit 231 sets the plurality of regions for monitoring, the body movement processing unit 230 (body movement analysis unit 233) analyzes the video of the surveillance camera during the imaging, and records whether the examination area remains in the first region, whether the examination area has protruded into the second region, a time during which the examination area remains in the first region or remains in the first region after returning to the first region, and a time during which the examination area remains in the second region (S14).

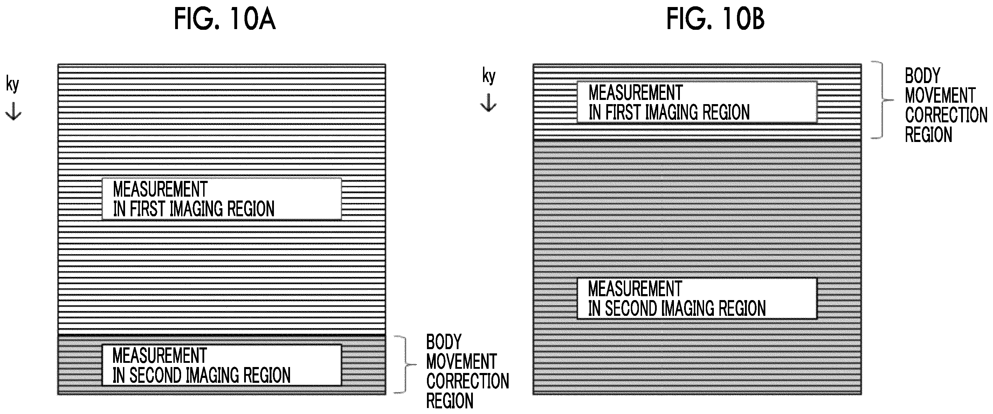

Here, in a case where the subject has moved and the examination area moves out of the first region into the second region, the determination unit 235 compares the time during which the examination area is within the first region, that is, a measurement time in the first region, with the time during which the examination area remains in the second region, that is, a measurement time in the second region (S21). As a result, as shown in Fig. 10A, in a case where the measurement time in the first region is longer than the measurement time in the second region, the data collected in a case where the examination area has protruded into the second region is used as body movement correction target data (S22), and image reconstruction accompanied by the body movement correction is performed (S18). On the other hand, as shown in Fig. 10B, in a case where the measurement time in the first region is shorter than the measurement time in the second region, the data collected in a case where the examination area has protruded into the first region is set as the body movement correction target data (S23), and the image reconstruction accompanied by the body movement correction is performed (S18).

Regarding the method of the body movement correction, as in Embodiment 1, an appropriate method is adopted in consideration of the ratio of the body movement correction target data occupying the k-space and the disposition on the k-space.

Although not shown in Fig. 8, as shown in Fig. 9C, in a case where a third region is set outside the second region, the measurement time in each region may be compared, and data other than the data measured in the region having the longest measurement time may be set as the body movement correction target. In addition, in a case where the measurement time in each region is shorter than a predetermined time, it is possible to determine that it is difficult to perform effective body movement correction, and to perform a notification prompting the re-measurement or to automatically execute the re-measurement in the same manner as in the re-measurement of Embodiment 1 (Fig. 5: S17).

According to the present embodiment, by setting the plurality of regions around the examination area, it is possible to know the direction of the body movement of the subject, to provide appropriate feedback to the subject, the examination technician, and the like, and to quickly return to the initial position. As a result, the amount of body movement correction target data can be reduced, and effective body movement correction can be performed. In addition, by setting double or triple surrounding regions, it is possible to grasp in which direction and to what extent the body movement occurs, and it is possible to secure data that can be effectively used even in a case where the examination area moves, that is, data that can be used without the body movement correction, thereby reducing the need for re-examination due to the body movement.

### Other Embodiments

In the above-described embodiment, the image recognition or the feature amount extraction algorithm has been exemplified as the method of recognizing the examination area in a case of setting the region on the camera image, but it is also possible to use a known skeletal detection technology instead of or in addition to the image recognition or the feature amount extraction algorithm. The skeletal detection technology is a technology of detecting a skeleton of a human body based on body feature points, for example, the ears and jaw for the head, and the shoulders, elbows, wrists, knees, and ankles for the torso. For example, in a case where the examination area is the chest or abdomen, it is possible to set a target region in a part that is the chest or abdomen based on the skeletal structure detected using both shoulders and hip joints. The skeletal detection technology can also be used in a case where the body movement analysis unit analyzes the body movement to detect and analyze the movement of the examination area beyond the region based on changes in skeletal positions.

In addition, in the above-described embodiment, the case where the target region is set and the body movement is analyzed using the video from the surveillance camera 80 attached to the gantry has been described, but it is also possible to use a plurality of optical imaging devices such as a stereo camera instead of the surveillance camera or in addition to the surveillance camera. In the analysis using the image of the surveillance camera, it is possible to capture the movement of the image in a plane, but it is difficult to capture the movement of the image in a depth direction. On the other hand, with a stereo camera, an angular difference (parallax) from the camera to an object is calculated based on a pixel offset between two images, and a distance to the object, that is, depth information can be acquired by using the inter-camera distance and the parallax. Therefore, by using the surveillance camera 80 and the stereo camera in combination, it is possible to obtain three-dimensional information on the magnitude and the direction of the body movement, and it is possible to detect the body movement with higher accuracy.

Although the embodiments of the MRI apparatus according to the present invention have been described above, the present invention is characterized by setting and analyzing regions (two-dimensional regions) for monitoring the body movement, and adjusting the body movement correction based on the movement between the regions, and the present invention is not limited to these embodiments. An MRI apparatus including additional elements and functions and alternative elements and functions is also included in the present invention.

### Explanation of References

10: imaging unit
20: processor
210: imaging control unit
220: image generation unit
230: body movement processing unit
231: region setting unit
233: body movement analysis unit
235: determination unit

## Claims

1. A magnetic resonance imaging apparatus comprising:
an imaging unit that collects a nuclear magnetic resonance signal generated from a subject;
an image generation unit that generates an image using the nuclear magnetic resonance signal; and
a processor that is configured to acquire a video from an imaging device which detects a body movement of the subject and process body movement information of the subject,
wherein the processor is configured to set a first imaging region for monitoring an examination area of the subject in the video, determine at least one of presence or absence of the body movement, duration of the body movement, a direction of the body movement, or a magnitude of the body movement based on whether or not the examination area is shifted from the first imaging region to an outside of the first imaging region, and control body movement correction by the image generation unit.

2. The magnetic resonance imaging apparatus according to claim 1,
wherein the processor is configured to compare a period during which the examination area remains outside the first imaging region with a predetermined threshold to determine the duration of the body movement.

3. The magnetic resonance imaging apparatus according to claim 2,
wherein the processor is configured to, in a case where the period during which the examination area remains outside the first imaging region is equal to or shorter than the predetermined threshold, designate a nuclear magnetic resonance signal collected by the imaging unit during the period as body movement correction data in a case where the image generation unit generates the image.

4. The magnetic resonance imaging apparatus according to claim 2,
wherein the processor is configured to, in a case where the period during which the examination area remains outside the first imaging region exceeds the predetermined threshold, notify a user.

5. The magnetic resonance imaging apparatus according to claim 2,
wherein the predetermined threshold is equal to or less than a time taken until the image generation unit collects a nuclear magnetic resonance signal capable of constructing an image.

6. The magnetic resonance imaging apparatus according to claim 5,
wherein the predetermined threshold is equal to or less than a time required to collect 20% or more of k-space data consisting of the nuclear magnetic resonance signal collected by the imaging unit.

7. The magnetic resonance imaging apparatus according to claim 2,
wherein the processor is configured to change the predetermined threshold according to a repetition time (TR) in an imaging sequence performed by the imaging unit.

8. The magnetic resonance imaging apparatus according to any one of the preceding claims,
wherein the first imaging region is a region including the examination area.

9. The magnetic resonance imaging apparatus according to any one of the preceding claims,
wherein the processor is configured to set a second imaging region outside the first imaging region, and determine from the video that the examination area has moved outside the first imaging region in a case where the examination area has moved to a second imaging region side.

10. The magnetic resonance imaging apparatus according to claim 9,
wherein the processor is configured to set a gap between the first imaging region and the second imaging region.

11. The magnetic resonance imaging apparatus according to claim 9,
wherein the processor is configured to set the second imaging region to a region obtained by extending the first imaging region in a one-dimensional direction.

12. The magnetic resonance imaging apparatus according to claim 9,
wherein the processor is configured to set the second imaging region to a region obtained by extending the first imaging region in a two-dimensional direction.

13. The magnetic resonance imaging apparatus according to claim 9,
wherein the second imaging region includes an inner region close to the first imaging region and an outer region outside the inner region.

14. The magnetic resonance imaging apparatus according to any one of the preceding claims,
wherein the processor is configured to acquire videos from a plurality of imaging devices having different video acquisition positions, and determine a body movement of the examination area using the videos.

15. The magnetic resonance imaging apparatus according to any one of the preceding claims,
wherein the processor is configured to determine the body movement by feature point analysis or skeletal analysis in the video.
